# DEMANDE DE BREVET EUROPEEN

(11) **EP 4 124 652 A1**
(43) Date de publication de la demande: **01.02.2023**
(21) Numéro de dépôt: 21306041.1
(22) Date de dépôt: 27.07.2021
(51) Int. Cl.: C12M 1/107, C12M 1/00, C12M 1/02

(54) **DISPOSITIF DE TRAITEMENT D'INTRANTS BI-ÉTAGÉ ET PROCÉDÉ DE TRAITEMENT D'INTRANTS MIS EN OEUVRE DANS UN TEL DISPOSITIF**

(71) Demandeur: Syctom L'Agence Metropolitaine Des Dechets Menagers, 75013 Paris (FR); Syndicat Interdepartemental pour l'Assainissement de l'Agglomeration Parisienne, 75012 Paris (FR)
(72) Inventeur: MOREAU, Thomas, 78190 TRAPPES (FR); MAGIS, Alain, B - 4682 HOUTAIN SAINT SIMEON (BE); ANDRE, Laura, 60360 LIHUS (FR); RIBEIRO, Thierry, 80250 AILLY SUR NOYE (FR); PAUSS, André, 60200 COMPIEGNE (FR); BILLETTE, Patrick, 92500 RUEIL MALMAISON (FR); ROUTHIER, Florian, 92000 NANTERRE (FR); BRUNET, Antoine, 92000 NANTERRE (FR)
(74) Mandataire: Plasseraud IP

(57) **Abrégé**

La présente invention appartient au domaine de la production de méthane, plus particulièrement de la valorisation d'intrants comprenant un mélange d'une fraction organique des déchets ménagers, des boues et éventuellement de fumiers et des graisses.

## Description

La présente invention appartient au domaine de la production de méthane, plus particulièrement de la valorisation d'intrants organiques, en particulier et de préférence comprenant un mélange d'une fraction organique des déchets ménagers, des boues et éventuellement de fumiers et des graisses.

L'objet de la présente invention se rapporte à un dispositif de dispositif de traitement d'intrants, comprenant : un premier et un second réacteur infiniment mélangés en série, ledit second réacteur étant muni d'une boucle de recirculation comprenant une unité de traitement par hydrolyse thermique comprenant un compartiment de traitement par détente flash, ladite boucle étant relié d'une part à la sortie du second réacteur et d'autre part à l'entrée du second réacteur et à un procédé de traitement d'intrants comprenant une étape d'hydrolyse en conditions thermophiles, une étape de méthanogène, une fraction du digestat étant ensuite soumise à une détente flash avant d'être réinjectée dans le second réacteur.

Des procédés et dispositifs de traitement d'intrants sont connus :
- L'hydrolyse enzymatique permet une dégradation accélérée de la matière, mais elle nécessite l'injection d'enzymes dont la conservation est complexe : le coût de fonctionnement est donc important ce qui nuit à la rentabilité du procédé.
- L'utilisation de champs électriques pulsés est également à l'étude pour assurer une déstructuration poussée de la matière en vue de favoriser sa transformation au sein du procédé de méthanisation. Elle entraîne toutefois une forte consommation électrique et des résultats peu probants au regard de l'augmentation de rendement du procédé.
- L'utilisation d'une hydrolyse thermique en tête de procédé est également une solution développée par différentes entités et donnant de bons résultats. Elle est toutefois placée en tête de traitement et non sur une boucle de recirculation : la consommation énergétique est donc plus importante et le rendement global, bien qu'amélioré, reste inférieur à celui de la solution développée.

Il existe ainsi un besoin d'augmenter significativement les rendements des procédés de méthanisation, mais aussi de permettre la mise en œuvre desdits procédés sur des mélanges d'intrants comprenant un mélange d'une fraction organique des déchets ménagers, des boues et/ou des graisses, ce qui n'est actuellement pas possible avec les procédés connus.

Il est du mérite de la demanderesse d'avoir développé un nouveau dispositif et un nouveau procédé associé qui pallient les défauts et contraintes relevés dans les dispositifs et procédés connus.

L'invention se rapporte ainsi à un dispositif de traitement d'intrants, comprenant :
- un premier réacteur infiniment mélangé ;
- un second réacteur infiniment mélangé, ledit second réacteur étant muni d'une boucle de recirculation comprenant une unité de traitement par hydrolyse thermique comprenant un compartiment de traitement par détente flash, ladite boucle étant reliée d'une part à la sortie du second réacteur et d'autre part à l'entrée du second réacteur,
les premier et second réacteurs étant agencés en série.

On entend par « intrants », un mélange d'une fraction organique de déchets ménagers, de boues, de fumiers et/ou des graisses. De préférence, il s'agit d'une fraction organique de déchets ménagers et de boues. Les déchets ménagers sont généralement issus de centres de traitement équipés d'une unité de criblage permettant l'extraction de la fraction dont le diamètre est compris dans l'intervalle allant de 0 à 10 mm riche en matière organique. Les boues sont généralement issues de stations de traitement des eaux usées et peuvent comprendre des boues primaires issues de la décantation des eaux ou des boues biologiques issues du traitement aérobie. Les intrants peuvent également comprendre des fumiers, généralement issus de centres équins. Les intrants ont généralement une siccité de 6 à 7 %.

On entend par « relié », une connexion directe ou indirecte entre deux éléments du dispositif.

On entend par « unité de traitement par hydrolyse thermique comprenant un compartiment de traitement par détente flash » une unité composée d'un réacteur de mise en pression avec injection de vapeur et d'un réacteur de détente flash munie d'une vanne pour mise à l'atmosphère. L'hydrolyse thermique se déroule dans le réacteur de mise en pression, sous l'effet de la pression et de la température. Ainsi, dans ladite unité de traitement, la mise en pression et la détente flash peuvent être réalisée dans deux réacteurs séparés. Alternativement, la mise en pression et la détente flash peuvent être réalisée dans un seul et même réacteur remplissant les deux fonctions.

Avantageusement, le premier réacteur est apte à fonctionner en conditions thermophiles. De préférence, le premier réacteur est configuré pour fonctionner en conditions thermophiles. On entend par « conditions thermophiles », des conditions permettant à des organismes thermophiles de prospérer. Les organismes thermophiles peuvent vivre et se multiplier entre 50 et 60 °C.

Avantageusement, le premier réacteur est un réacteur infiniment mélangé fonctionnant en continu ou CSTR en anglais pour Continuous Stirred-Tank Reactor. Les dimensions du premier réacteur sont généralement configurées pour avoir un rapport hauteur/diamètre de 1,5. De préférence, le premier réacteur est de forme cylindrique s'étendant autour d'un axe d'extension sensiblement vertical. Le réacteur peut présenter une section transversale par rapport à l'axe d'extension. La section transversale peut être de forme diverse ; par exemple de forme circulaire, rectangulaire ou carrée. Le premier réacteur comprend au moins une entrée et au moins une sortie. Le premier réacteur comprend une partie supérieure et une partie inférieure. De préférence, le premier réacteur est configuré pour fonctionner en conditions thermophiles. Le premier réacteur peut être muni d'un système de régulation de température (de préférence de chauffage) permettant d'assurer une homéostasie à 55 °C environ et isolé thermiquement. Il peut s'agir par exemple d'un système de régulation de température par recirculation et d'une isolation thermique poussée. Le système de régulation de température peut être un échangeur tubulaire ou spiralé. Le premier réacteur peut comprendre en outre une deuxième sortie (pour la récupération du gaz produit lors de l'étape d'hydrolyse). La deuxième sortie peut être munie d'un débitmètre. La deuxième sortie peut être reliée à un gazomètre. Il est ainsi possible de recueillir le gaz produit lors de l'étape d'hydrolyse dans le premier réacteur lors de la mise-en-œuvre du procédé selon l'invention. Le gazomètre peut être de forme sphérique et fixé sur le sol ou présenter la forme d'une demi-sphère, par exemple lorsqu'il est fixé sur la partie supérieure du premier réacteur.

Avantageusement, le second réacteur est apte à fonctionner en conditions mésophiles. De préférence, le second réacteur est configuré pour fonctionner en conditions mésophiles. On entend par « conditions mésophiles », des conditions permettant à des organismes mésophiles de prospérer (température de 37 °C à pH neutre). Un organisme mésophile est une forme de vie qui prospère au mieux dans des conditions de température modérée. Classiquement, on parle de bactéries mésophiles lorsqu'elles vivent à des températures comprises entre 20 et 40 °C.

Avantageusement, le second réacteur est un réacteur infiniment mélangé fonctionnant en continu ou CSTR en anglais pour Continuous Stirred-Tank Reactor. Les dimensions du second réacteur sont généralement configurées pour avoir un rapport hauteur/diamètre de 1,5. De préférence, le second réacteur est de forme cylindrique s'étendant autour d'un axe d'extension sensiblement vertical. Le réacteur peut présenter une section transversale par rapport à l'axe d'extension. La section transversale peut être de forme diverse ; par exemple de forme circulaire, rectangulaire ou carrée. Le second réacteur comprend au moins une entrée et au moins une sortie. Le second réacteur comprend une partie supérieure et une partie inférieure. De préférence, le second réacteur est configuré pour fonctionner en conditions mésophiles. Le second réacteur peut être muni d'un système de régulation de température. Il peut s'agir par exemple d'un système de régulation de température par recirculation et d'une isolation thermique poussée. Le système de régulation de température par recirculation peut être un échangeur tubulaire ou spiralé. Le second réacteur peut comprendre en outre une deuxième sortie (pour la récupération du gaz produit lors de l'étape de méthanogénèse). La deuxième sortie peut être munie d'un débitmètre. La deuxième sortie peut être reliée à un gazomètre. Il est ainsi possible de recueillir le méthane produit dans le second réacteur lors de la mise-en-œuvre du procédé selon l'invention. Le gazomètre peut être de forme sphérique et fixé sur le sol ou présenter la forme d'une demi-sphère, par exemple lorsqu'il est fixé sur la partie supérieure du second réacteur.

Avantageusement, le second réacteur est muni d'une boucle de recirculation, ladite boucle étant reliée d'une part à la sortie du second réacteur et d'autre part à l'entrée du second réacteur. Le sens de circulation prévu dans la boucle de recirculation allant de la sortie du second réacteur vers l'entrée du second réacteur. La boucle de recirculation comprend une unité de traitement par hydrolyse thermique comprenant un compartiment de traitement par détente flash.

Avantageusement, l'unité de traitement par hydrolyse thermique comprenant un compartiment de traitement par détente flash comprend un réacteur de détente flash composée d'un réacteur de mise en pression avec injection de vapeur puis d'un réacteur de détente munie d'une vanne pour mise à l'atmosphère. La boucle de recirculation peut comprendre en outre une presse à vis située en amont du réacteur de mise en pression avec injection de vapeur de l'unité de traitement par hydrolyse thermique, par rapport au sens de circulation dans la boucle et permettant de limiter la teneur en eau du mélange (siccité cible de 12 %) et donc la consommation énergétique nécessaire au chauffage du mélange.

Avantageusement, le(s) premier et/ou second réacteur(s) peut/peuvent comprendre un agitateur mécanique. L'agitateur mécanique est de préférence pendulaire.

Avantageusement, les premier et second réacteurs sont agencés en série, c'est-à-dire que l'entrée de second réacteur est reliée à la sortie du premier réacteur. Les sens de circulation des intrants étant prévus dans le sens premier puis second réacteur. La sortie du premier réacteur est reliée à l'entrée du second réacteur. L'entrée du second réacteur est reliée à la sortie du premier réacteur et à la sortie de l'unité de traitement par détente flash comprise dans la boucle de recirculation. La sortie du second réacteur est reliée à l'unité de traitement par détente flash et à l'évacuation du digestat en excès.

Les premier et second réacteurs (hors boucle de recirculation comprenant l'unité de traitement par détente flash) peuvent être identiques ou différents.

Avantageusement, le dispositif selon l'invention peut comprendre en outre un moyen d'homogénéisation. Le moyen d'homogénéisation comprend au moins deux entrées et une sortie. Le moyen d'homogénéisation peut ainsi être relié à la sortie du premier réacteur et à la boucle de recirculation. Le moyen d'homogénéisation peut être un mélangeur. Il peut s'agir d'une cuve munie d'un agitateur mécanique, de préférence un agitateur pendulaire. Le moyen d'homogénéisation peut être relié à l'entrée du second réacteur. Le moyen d'homogénéisation permet ainsi d'homogénéiser le mélange digestat A/digestat C avant son introduction dans le second réacteur, selon le procédé de l'invention décrit ci-dessous.

Avantageusement, le dispositif selon l'invention peut comprendre un moyen de stockage. Le moyen de stockage (également appelé cuve tampon) peut être positionné en amont du premier réacteur, entre le premier et le second réacteur et/ou entre le second réacteur et la sortie et/ou la boucle de recirculation. Le moyen de stockage peut être une cuve dite « tampon », permettant de réguler les transferts de masse d'un réacteur à un autre, sur la boucle ou en sortie du dispositif, afin d'éviter tout débordement ou colmatage. Lorsque le moyen de stockage est positionné entre le premier et le second réacteur, il peut être identique ou différent du moyen d'homogénéisation.

Avantageusement, le dispositif selon l'invention peut comprendre en outre un échangeur thermique, de préférence tubulaire. L'échangeur thermique peut être relié à l'unité de traitement par hydrolyse thermique comprenant un compartiment de traitement par détente flash et plus particulièrement directement au compartiment de détente flash. L'échangeur thermique peut en outre être relié au système de régulation de température du premier réacteur ou au système de régulation de température du second réacteur. L'échangeur thermique peut permettre de valoriser la chaleur fatale de l'équipement. On entend par « chaleur fatale » ou « chaleur de récupération », la chaleur générée par le compartiment de détente flash qui n'en constitue pas la finalité première, et qui n'est normalement pas récupérée. Il s'agit ici par exemple de transférer les calories récupérées sur l'unité de traitement par hydrolyse thermique comprenant un compartiment de traitement par détente flash et de les utiliser pour réguler la température du premier et/ou du second réacteur.

Avantageusement, le dispositif selon l'invention peut être muni d'un ensemble de pompes et de vannes permettant le transfert automatique ou manuel des digestats A, B et/ou C d'un élément du dispositif à un autre.

L'invention se rapporte également à un procédé de traitement d'intrants, comprenant les étapes :
a) introduction d'intrants dans un premier réacteur infiniment mélangé ;
b) hydrolyse en conditions thermophiles du mélange d'intrants dans le premier réacteur infiniment mélangé, de manière à obtenir un digestat A ;
c) introduction dans un second réacteur infiniment mélangé du digestat A obtenu à l'étape b) et du digestat C obtenu à l'étape e) ;
d) méthanogénèse en conditions mésophiles du digestat A et du digestat C introduits dans le second réacteur à l'étape c), de manière à obtenir du méthane et un digestat B;
e) séparation du digestat B obtenu à l'étape d) en une première fraction (F1) et seconde fraction (F2), ladite première fraction (F1), optionnellement épaissie, étant soumise à une hydrolyse thermique suivi d'une détente flash de manière à obtenir un digestat C, ledit digestat C étant réintroduit dans le second réacteur à l'étape c).

Lors de la mise en route du procédé selon l'invention, il est possible que seul du digestat A soit introduit dans le second réacteur à l'étape c) (car il n'existe pas encore de digestat C), afin de produire du digestat B dont une fraction sera transformée en digestat C. À l'issue de cette mise en route du procédé, l'étape c) est réalisée normalement, comme indiqué ci-dessus. Lors d'une mise en route du procédé selon l'invention, le digestat C peut aussi provenir d'une précédente mise en œuvre du procédé, dans ce cas de figure, même lors de la mise en route du procédé, un digestat A et un digestat C sont introduits lors de l'étape c).

Le procédé selon l'invention permet ainsi une hydrolyse plus complète de la matière. Le procédé selon l'invention permet de dégrader de la matière organique difficilement dégradable qui subsiste dans le digestat et la transformer en molécules facilement assimilables par la flore bactérienne. Il s'agit par exemple de longues chaînes carbonées (ayant généralement de 10 à 30 atomes de carbone) qui n'étaient généralement pas dégradées dans les procédés connus. Le produit obtenu à l'issue du procédé est tel, qu'il permet d'assurer une hydrolyse physique de la matière organique réfractaire : les longues chaînes carbonées alors sont découpées en morceaux par l'effet de la chaleur et de la pression lors de la détente flash et deviennent plus facilement mobilisables par la flore bactérienne.

Le procédé selon l'invention permet de convertir la matière organique hydrolysée thermiquement en méthane (à l'étape b)), et d'atteindre un taux global d'expression du BioMéthane Potentiel (BMP) des intrants de 110 %.

Avantageusement, lors de la mise en œuvre de l'étape a), les intrants peuvent être introduits dans le premier réacteur au moyen d'une pompe, de préférence une ou plusieurs pompes péristaltiques. Préalablement à la mise en œuvre de l'étape a), les intrants peuvent subir une étape de broyage. Le broyage peut être réalisé au moyen de couteaux ; permettant de réduire la taille des fibres longues (par exemple les fibres mesurant plus de 5 cm).

Avantageusement, le procédé peut être en œuvre par batch (ou lot), en continu ou en semi-continu. Ainsi les étapes a) à e) peuvent être mise en œuvre consécutivement ou simultanément.

Avantageusement, l'étape b) d'hydrolyse est réalisée en conditions thermophiles dans le premier réacteur. Le temps de séjour de l'étape b) peut être compris dans un intervalle allant de 2 à 10 jours, de préférence 5 jours (on entend par jour, une durée de 24 heures). La température de l'hydrolyse de l'étape b) peut être comprise dans un intervalle allant de 50 à 60 °C, de préférence 55 °C. L'étape b) peut comprendre une agitation pendant tout ou partie de la réaction d'hydrolyse. À l'issue de l'étape b), on obtient un digestat A. Le digestat A est généralement composé d'un mélange d'acides gras volatiles (AGV) en forte concentration. Il peut s'agir par exemple d'acide butyrique, mais aussi acide propionique ou acétique.

Avantageusement, lors de la mise en œuvre de l'étape c), le digestat A peut être introduit dans le second réacteur au moyen d'une pompe, de préférence une pompe péristaltique.

Avantageusement, lors de la mise en œuvre de l'étape c), le digestat C peut être introduit dans le second réacteur au moyen d'une pompe, de préférence une pompe péristaltique. Préalablement à la mise en œuvre de l'étape c), le digestat C peut subir une étape d'épaississement par presse à vis visant à augmenter la teneur en matière sèche du mélange. La phase d'épaississement permet l'atteinte d'une siccité cible de 12 %.

On définit un ratio volumique digestat C/digestat A. Dans l'étape c), le ratio V_{digestat C}/V_{digestat A} est compris dans un intervalle allant de 20 à 30 %.

Avantageusement, l'étape c) peut comprendre une sous étape de mélange et d'homogénéisation des digestats A et C avant leur introduction (sous forme de mélange) dans le second réacteur.

Avantageusement, l'étape d) de méthanogénèse est réalisée en conditions mésophiles dans le second réacteur. Le temps de séjour de l'étape d) peut être compris dans un intervalle allant de 10 à 20 jours, de préférence 15 jours. La température de la méthanogénèse de l'étape d) peut être comprise dans un intervalle allant de 34 à 40 °C, de préférence 37 °C. L'étape d) peut comprendre une agitation pendant tout ou partie de la méthanogénèse. À l'issue de l'étape d), on obtient un digestat B. Le digestat B est généralement composé de matières minérales issues de la dégradation de la matière organique.

Avantageusement, l'étape d) de méthanogénèse peut comprendre en outre une sous-étape de contrôle du pH et de régulation du pH. Généralement, dans ce type de réaction, un pH < 6 indique un empoisonnement du milieu réactionnel (acidose) et une perte de productivité. L'étape d) peut ainsi comprendre une sous-étape de mesure du pH et de régulation du pH (automatique ou manuel), par ajout d'une base (généralement de la soude), lorsque la valeur du pH mesuré est inférieure ou égale à 6, de préférence inférieure ou égale à 6,5.

Le méthane est produit lors de l'étape d), et plus particulièrement lors de la méthanogénèse. Le méthane peut être recueilli dans un gazomètre puis extrait via le gazomètre pour être valorisé.

Avantageusement, l'étape e) comprend la séparation du digestat B obtenu à l'étape d), en deux fractions F1 et F2, ladite fraction F1, optionnellement épaissie, étant soumise à une hydrolyse thermique suivi d'une détente flash de manière à obtenir un digestat C. Ledit digestat C est réintroduit dans le second réacteur à l'étape c). La séparation de l'étape e) peut être réalisée au moyen d'une presse à vis. À l'issue de la séparation de l'étape e) on obtient deux fractions : une première fraction (F1) qui est optionnellement épaissie avant d'être soumise à une hydrolyse thermique suivi d'une détente flash de manière à obtenir un digestat C ; et une seconde fraction (F2), qui n'est pas épaissie, ni soumise à une hydrolyse thermique suivi d'une détente flash et qui est le digestat D, produit final obtenu à l'issue du procédé selon l'invention. On définit ainsi un ratio massique m_{F1}/m_{F2}. Le ratio m_{F1}/m_{F2} peut être compris dans un intervalle allant de 25 à 35 %, de préférence 30%. L'étape e) peut comprendre une sous-étape de stockage avant la séparation.

Avantageusement, la fraction (F1) séparée lors de l'étape e) représente 30 à 40 % du volume de digestat A introduit dans le second réacteur à l'étape c).

Avantageusement, l'épaississement de la première fraction (F1) de l'étape e), peut être réalisé dans une presse à vis. L'épaississement est optionnel et peut être mis en œuvre jusqu'à l'obtention d'une siccité 10 à 14 %, de préférence 12%. On entend par « siccité », la teneur en matière sèche du mélange considéré.

Avantageusement, le traitement par l'hydrolyse thermique suivi de détente flash de l'étape e) est mise en œuvre dans l'unité de traitement par hydrolyse thermique comprenant un compartiment de traitement par détente flash décrite précédemment dans le cadre de la présente invention. L'hydrolyse thermique suivie de détente flash de l'étape e) peut comprendre les sous-étapes :
i) introduction et mise sous pression dans un réacteur de mise en pression de la première fraction F1 par injection de vapeur dans le réacteur, à une pression allant de 3 à 12 bars, de préférence de 4 à 5 bars, à une température allant de 140 à 190°C, pendant une durée allant de 20 à 40 minutes,
ii) retour à la pression atmosphérique de la fraction de co-digestat B dans le susdit réacteur de mise en pression ou dans un autre réacteur, un réacteur de détente flash, et obtention du digestat C.

Avantageusement, l'étape ii) de retour à la pression atmosphérique est quasi instantanée. Il est réalisé au moyen d'une vanne, de préférence une vanne de détente à modulation.

Avantageusement, l'hydrolyse thermique suivi de détente flash de l'étape e) peut comprendre en outre une étape iii) de transfert de calorie du digestat C pour chauffer la température des intrants ou réguler la température du premier et/ou du second réacteur, de préférence au moyen d'un échangeur thermique tubulaire.

Avantageusement, le procédé selon l'invention peut comprendre en outre une étape f) de déshydratation de la fraction F2 (digestat D), suivie d'un séchage.

L'invention présente les avantages suivants, non limitatifs :
- amélioration du rendement, meilleure production de méthane pour une même quantité de matière d'intrants traitée ;
- un épaississement en amont du traitement par une hydrolyse qualifiée d'hydrolyse thermique (étape e)) permet de limiter la consommation d'énergie thermique du système et améliore donc sa rentabilité énergétique ;
- un épaississement en amont du traitement par méthanogénèse (digestat C) permet un apport de matière plus concentrée dans le second réacteur mésophile et permet d'utiliser une charge appliquée supérieure et donc de limiter les besoins en volume utile ;
- amélioration significative des performances de digestion notamment grâce à la réduction du digestat B par solubilisation d'une partie des matières en suspension lors de la détente flash, stérilisation du digestat dans le réacteur de pressurisation de la détente flash, une diminution de la viscosité du digestat B octroyant un gain d'énergie au niveau du pompage en général et de l'agitation du réacteur thermophile et mésophile, amélioration de la biodégradation des intrants avec une augmentation importante du BMP ;
- augmentation de la production de biogaz (méthane) de plus de 20 % du BMP mesuré selon la norme en vigueur. ;
- amélioration de la déshydratation de la fraction solide des digestats de la fraction F2.

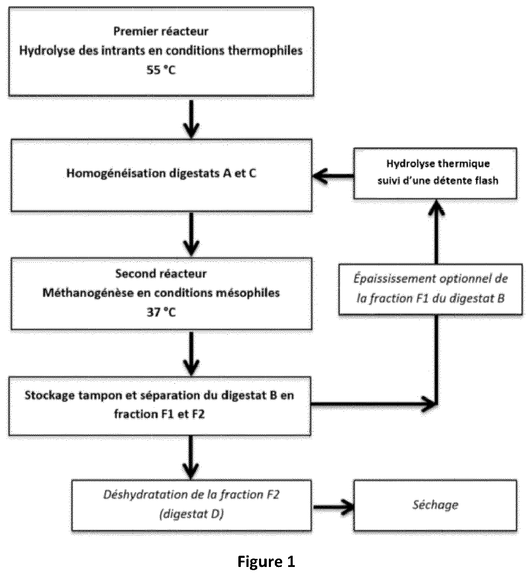

La figure 1 représente un exemple de schéma fonctionnel de mise en œuvre du procédé et l'enchaînement logique des étapes a) à e).

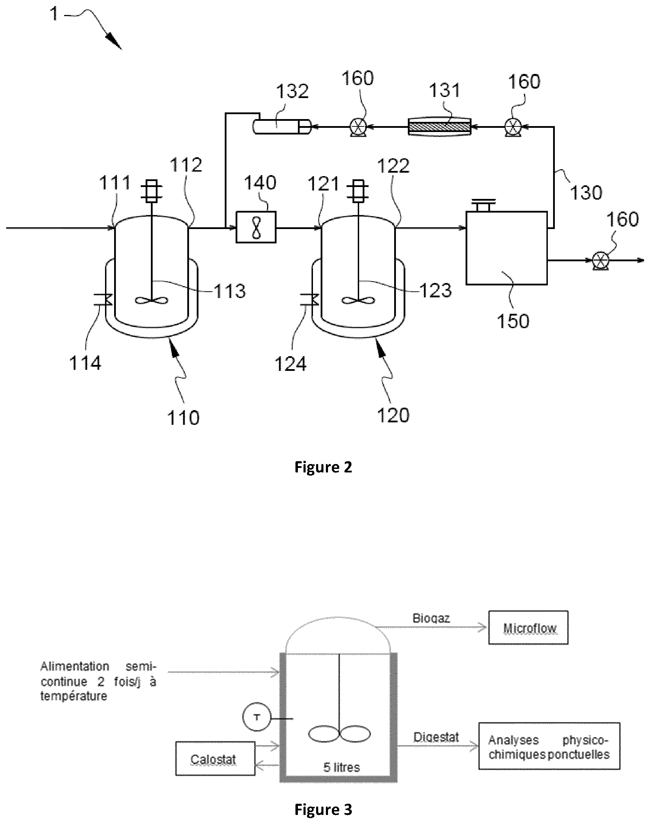

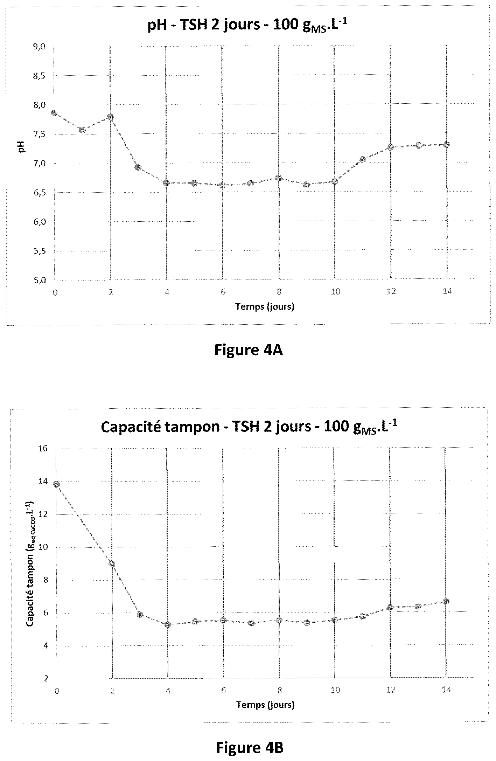

La figure 2 représente un dispositif 1 selon l'invention, comprenant un premier réacteur 110 comprenant une entrée 111, une sortie 112, un agitateur mécanique 113 et un système de chauffage 114, un second réacteur 120 comprenant une entrée 121, une sortie 122, un agitateur mécanique 123 et un système de chauffage 124, une boucle de recirculation 130 munie d'une presse à vis 131 et d'une unité de traitement par hydrolyse thermique comprenant un compartiment de traitement par détente flash 132, un moyen d'homogénéisation 140, un moyen de stockage (cuve tampon) 150 et un ensemble de pompes 160.La figure 3 représente le dispositif réactionnel utilisé pour les réacteurs 110 et 120.Les figures 4A à 4F représentent le suivi des paramètres clés du procédé au niveau du réacteur 110 : (4A) Suivi du pH en fonction du temps pour plusieurs temps de séjour de 2 jours à 100 g_{MS}.L⁻¹ ; (4B) Suivi de la capacité tampon en fonction du temps, pour plusieurs temps de séjour de 2 jours, 100 g_{MS}.L⁻¹ ;
(4C) Suivi des AGV en fonction du temps, pour plusieurs temps de séjour de 2 jours, 100 g_{MS}.L⁻¹ ; (4D) Suivi du ratio AGV / TAC en fonction du temps, pour plusieurs temps de séjour de 2 jours, 100 g_{MS}.L⁻¹ ; (4E) Suivi flux relatif de biogaz en fonction du temps, pour plusieurs temps de séjour de 2 jours, 100 g_{MS}.L⁻¹ ; (4F) Suivi flux relatif de méthane en fonction du temps, pour plusieurs temps de séjour de 2 jours, 100 g_{MS}.L⁻¹.

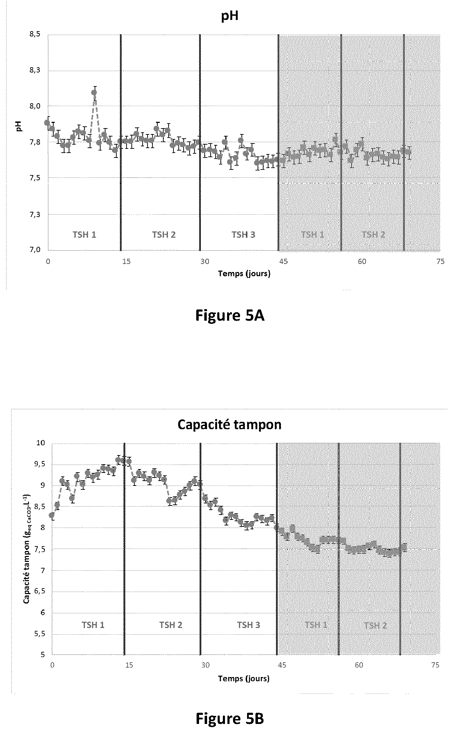

Les figures 5A à 5H représentent le suivi des paramètres clés du procédé au niveau du réacteur 120 : (A) Suivi du pH du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash ; (B) Suivi de la capacité tampon du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash ; (C) Suivi des AGV du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash ;
(D) Suivi du ratio AGV / TAC du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash ;
(E) Suivi de la composition en gaz (CH₄ et CO₂) du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash ;
(F) Suivi du flux relatif de biogaz du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash ;
(G) Suivi du flux de méthane du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash ;
(H) Suivi de la dégradation des MV et MVS du réacteur mésophile en fonction du temps, pour 3 temps de séjour de 15 jours sur du digestat A puis sur 2 temps de séjours de 15 jours (zone grise) sur un mélange de digestat A et digestat C issue de l'hydrolyse thermique et détente flash.

L'invention sera mieux comprise à la lecture des exemples, non limitatifs, qui suivent.

### Exemple 1 :

### A. Matériel et méthode

Le procédé de l'invention a été réalisé sur un mélange d'intrants représentatif constitué des éléments suivants (en matière sèche, MS) :
- 80 % de fraction organique de déchets ménagers (fraction 0 à 10 mm criblée, provenance Chagny) ;
- 20 % de boues biologiques non digérées (provenance usine Seine Aval) ;
- Concentration cible du mélange de 100 gMS/L.

Les boues traitées sont caractérisées par les paramètres spécifiques suivants :

**Tableau 1 : caractéristiques des boues traitées.**

| **Paramètre** | **Boues biologiques non digérées provenant de l'usine de Seine Aval (SIAAP)** |
|---|---|
| **Humidité (%)** | 95,4 |
| **Teneur en MS (%)** | 4,58 |
| **Teneur en MO (% MS)** | 84,6 |
| **DCO (g/gMS)** | 1,711 |
| **BMP expérimental (NLCH₄/kg_{MS})** | **292** |

La fraction organique de déchets ménagers (FOR) traitée est caractérisée par les paramètres spécifiques suivants :

**Tableau 2 : caractéristiques de la FOR traitée.**

| **Paramètre** | **Valeur** |
|---|---|
| **Humidité (%)** | 53,7 |
| **Teneur en MS (%)** | 46,31 |
| **Teneur en MO (% MS)** | 66,19 |
| **DCO (g/gMS)** | n.d. |
| **BMP expérimental (NLCH₄/kg_{MS})** | 217 |

Le procédé a été mis en œuvre les quantités suivantes :

**Tableau 3 : quantités mises en œuvre.**

| **Concentration essai** | **100** | **g/L** |
|---|---|---|
| Quantité de MS jour | 250,00 | g/jour |
| Pourcentage MS provenant de la FOR | 80 | % |
| Pourcentage MS provenant des boues | 20 | % |
| Quantité de MS provenant de la FOR | 200,00 | gMS/jour |
| Quantité de MS provenant des boues | 50,00 | gMS/jour |
| Concentration MS dans la FOR | 46 | % |
| Concentration MS boues | 45,80 | g/L réel |
| Matière brute nécessaire boues | 1,09 | litres boues/jour |
| Matière brute nécessaire FOR | 431,97 | gMBFOR/jour |
| Densité FOR | 0,55 | t/m³ |
| Volume FOR | 0,79 | litres FOR |
| Volume total intrants | 1,88 | litres |
| Complément eau pour atteinte concentration cible | 0,62 | litres |

| **Concentration cible (rappel)** | **100,00** | **g/L** |
|---|---|---|
| Matière brute totale boues pour essai | 6,55 | litres/essai |
| Matière brute totale FOR pour essai | 2,59 | kgMBFOR/essai |

Le premier réacteur (thermophile) est représenté sur la figure 3.

Les caractéristiques techniques du premier réacteur sont les suivantes :
- Volume utile du réacteur : 5 litres ;
- Système d'agitation à pales (CSTR) ;
- Dispositif de chauffage par double paroi avec système de régulation associé ;
- 1 port sortie gaz et 6 ports alimentation ;
- Débitmètre autonome Microflow (gamme de mesure de 20 à 4 000 mL/h) et système d'acquisition multivoies.

Le second réacteur (mésophile) est constitué des mêmes éléments que le premier réacteur et est placé en série.

Les caractéristiques techniques de l'unité de traitement par hydrolyse thermique comprenant un compartiment de traitement par détente flash sont les suivantes :
- un réacteur batch de mise en pression de 2,5 litres équipé d'un système de chauffage à l'huile thermique et d'un agitateur ;
- un réacteur de détente flash muni d'une vanne pour mise à l'atmosphère ;
- un dispositif de chauffage à l'huile thermique avec un maximum de 180 °C ;
- un système de régulation de température ;
- les tuyauteries de transfert, la robinetterie et l'instrumentation ad hoc.

### B. Résultats obtenus :

### a) Hydrolyse en conditions thermophiles :

L'hydrolyse est réalisée sur les intrants décrits ci-dessus. Le temps de séjour dans le premier réacteur est fixé à 2 jours. La température d'hydrolyse est de 55 °C.

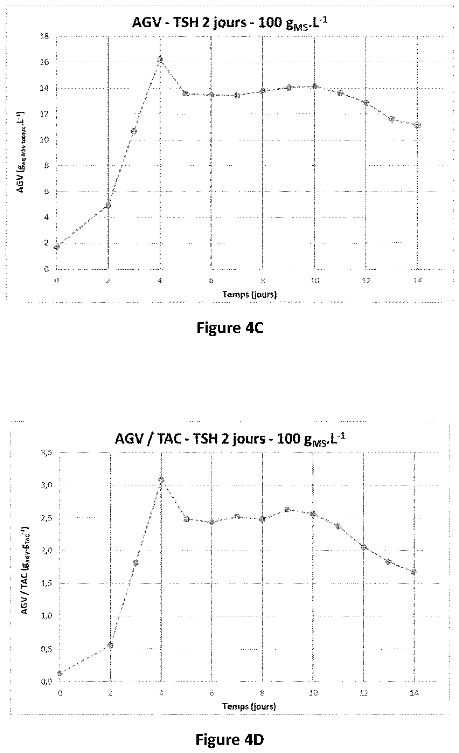

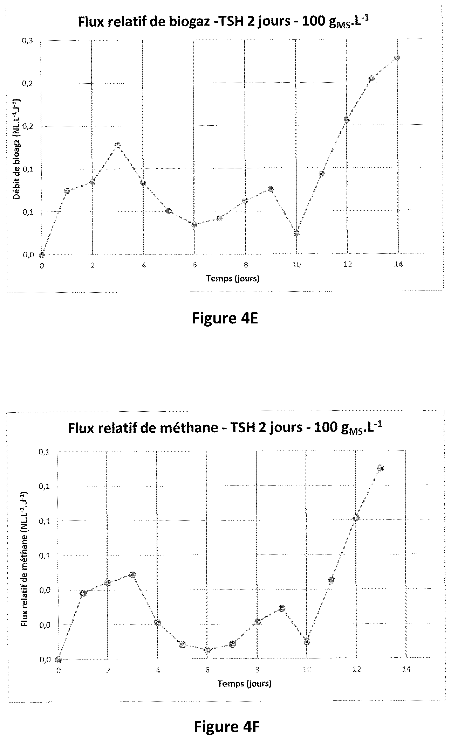

Le suivi de la réaction permet l'établissement des courbes présentées sur la figure 4. On observe une stabilisation du pH (Figure 4A) autour de 6,5 grâce à une consommation du pouvoir tampon qui atteint une asymptote à 6 g/L (Figure 4B). La production d'AGV augmente fortement (Figure 4C) et atteint 14 g/L. Le suivi de la production de biogaz (Figure 4E) et de sa teneur en méthane ((Figure 4F) indique une relativement faible production de biogaz par rapport au second réacteur, ce qui est cohérent avec l'étape d'hydrolyse recherchée. Le premier réacteur permet donc une hydrolyse efficace de la matière organique introduite.

Cette première étape permet d'obtenir un digestat A ayant une concentration en AGV de l'hydrolysat de 14 gAGV/L et un pouvoir tampon de 6 g/L. Le pH est stabilisé à une valeur de 6,5.

### b) Méthanogenèse en conditions mésophiles :

La méthanogénèse est réalisée dans un premier temps sur du digestat A obtenu après la réaction d'hydrolyse. Le réacteur de méthanogénèse permet d'atteindre un temps de séjour de 15 jours à une température stable de 37 °C.

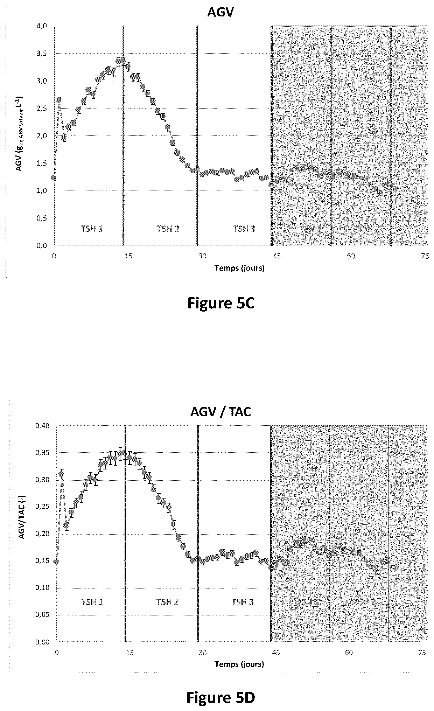

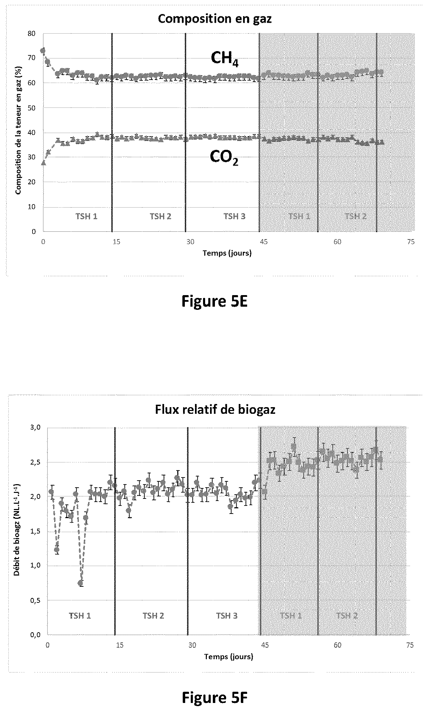

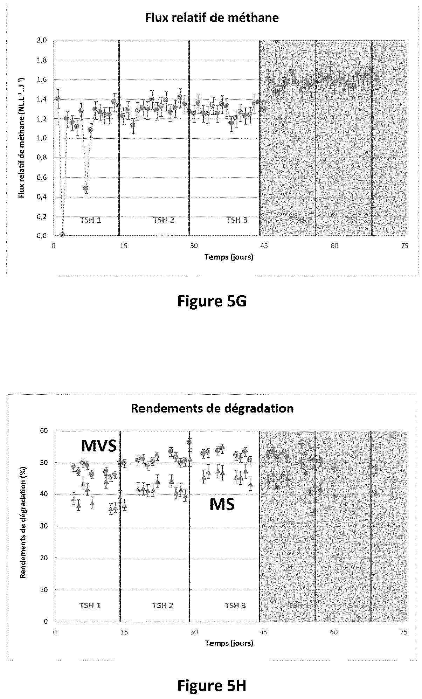

Les résultats de suivi des réacteurs sont présentés dans la figure 5. On observe un pH stable autour d'une valeur d'environ 7,5-8 (Figure 5A), une stabilisation de la capacité tampon à environ 6-7 g/L (Figure 5B) et une production de biogaz (Figure 5G) stabilisée avec une teneur en méthane de l'ordre de 65 % également stable (Figure 5F et Figure 5E). La production d'AGV (Figure 5C) se stabilise au bout de 2 THS et reste relativement faible au regard du premier réacteur, évitant ainsi la variation de pH susceptible d'inhibé l'activité bactérienne.

La productivité du système atteint 267 NL_{CH4}.kg_{MVS}⁻¹, soit 88,6 % de la valeur cible du BMP

Le second réacteur de méthanogenèse est ensuite alimenté avec un mélange de digestat A, obtenu après la réaction d'hydrolyse, et de digestat C, issu du traitement dans l'unité de détente flash d'un batch de digestat B obtenu précédemment. Le ratio MS_{digestat C}/MS_{digestat A} du mélange soumis à la méthanogénèse est égal à 30 %.

La méthanogénèse est réalisée sur un mélange de digestat A obtenu après la réaction d'hydrolyse, et de digestat C, issu du traitement dans l'unité de détente flash d'un batch précédent (165 °C, 5 bars). Le réacteur de méthanogénèse permet d'atteindre un temps de séjour de 15 jours à une température stable de 37 °C. Le ratio V_{digestat C}/V_{digestat A} du mélange soumis à la méthanogénèse est égal à 30 % en matière sèche.

Le suivi de la production biogaz (Figure 5F en zone grise) et de méthane (Figure 5G en zone grise) indique une réaction favorable et quasi instantanée du milieu à l'apport du nouveau mélange et une conservation du rendement de dégradation des MV et MVS (Figure 5H).

La productivité du réacteur mésophile atteint **331 NL_{CH4}.kg_{MVS}⁻¹, soit 110 % de la valeur cible du BMP.** Ainsi la comparaison directe avec le procédé témoin (digestat A uniquement) démontre les avantages du procédé selon l'invention.

### Exemple 2

Dans cet exemple, un procédé témoin (hors invention, pas de détente flash, MS_{digestat C}/MS_{digestat A} = 0) est comparé à la mise en œuvre du procédé selon l'exemple 1, dans laquelle les paramètres de détente flash ont été variés de la manière suivante : pression de 5 bars, température variant de 140 à 165 °C.

| | **SM (témoin digestat B)** | **Digestat C traité à 165 °C** | **Digestat C traité à 140 °C** | **Digestat C traité à 150 °C** |
|---|---|---|---|---|
| **Moyenne (Nm³_{CH4}.t_{MVS}⁻¹)** | 173,2 | 398,2 | 372,2 | 339,8 |
| **Minimum (Nm³_{CH4}.t_{MVS}⁻¹)** | 171,8 | 396,2 | 357,2 | 331,5 |
| **Maximum (Nm³_{CH4}.t_{MVS}⁻¹)** | 174,4 | 400,1 | 394,2 | 347,0 |
| **Écart-type (Nm³_{CH4}.t_{MVS}⁻¹)** | 1,3 | 2,8 | 19,5 | 7,8 |
| **CV (%)** | 0,8 | 0,7 | 5,2 | 2,3 |

Tableau 4 : comparaison des résultats obtenus du procédé mis en œuvre selon l'invention et d'un témoin (hors invention, sans hydrolyse thermique suivie de détente flash).

La caractérisation physico-chimique du digestat B et C est la suivante :

**Tableau 5 : Caractérisation physico-chimique du digestat B et C dans différentes conditions de traitement par détente flash.**

| **Témoin (digestat B non traité)** | | | | | |
|---|---|---|---|---|---|
| | **Minimum** | **Maximum** | **Moyenne** | **Écart-type** | **CV (%)** |
| **MS (%_{MB})** | 6,32 | 6,45 | 6,64 | 0,44 | 6,68 |
| **MVS (%_{MS})** | 58,7 | 63,3 | 61,6 | 2,5 | 4,1 |
| **MVS (%_{MB})** | 3,71 | 4,08 | 4,09 | 1,1 | 0,27 |
| **pH** | 7,64 | 7,76 | 7,72 | 0,07 | 0,85 |
| **NH₄⁺ (g.kg_{MB}⁻¹)** | 2,77 | 2,95 | 2,86 | 0,09 | 3,15 |

| **Digestat C traité à 140 °C** | | | | | |
|---|---|---|---|---|---|
| | **Minimum** | **Maximum** | **Moyenne** | **Écart-type** | **CV (%)** |
| **MS (%_{MB})** | 3,32 | 3,46 | 3,39 | 0,07 | 2,09 |
| **MVS (%_{MS})** | 66,1 | 66,4 | 66,2 | 0,2 | 0,3 |
| **MVS (%_{MB})** | 2,19 | 2,30 | 2,24 | 0,014 | 0,01 |
| **pH** | 9,79 | 9,81 | 9,80 | 0,01 | 0,11 |
| **NH₄⁺ (g.kg_{MB}⁻¹)** | 2,06 | 2,13 | 2,10 | 0,04 | 1,80 |

| **Digestat C traité à 150 °C** | | | | | |
|---|---|---|---|---|---|
| | **Minimum** | **Maximum** | **Moyenne** | **Écart-type** | **CV (%)** |
| **MS (%_{MB})** | 3,70 | 3,79 | 3,76 | 0,05 | 1,23 |
| **MVS (%_{MS})** | 54,5 | 66,4 | 66,4 | 0,1 | 0,1 |
| **MVS (%_{MB})** | 2,02 | 2,52 | 2,50 | 0,005 | 0,00 |
| **pH** | 9,62 | 9,65 | 9,64 | 0,01 | 0,15 |
| **NH₄⁺ (g.kg_{MB}⁻¹)** | 2,22 | 2,34 | 2,29 | 0,06 | 2,58 |

| **Digestat C traité à 165 °C** | | | | | |
|---|---|---|---|---|---|
| | **Minimum** | **Maximum** | **Moyenne** | **Écart-type** | **CV (%)** |
| **MS (%_{MB})** | 2,76 | 2,81 | 2,78 | 0,02 | 0,89 |
| **MVS (%_{MS})** | 72,0 | 72,3 | 72,1 | 0,1 | 0,2 |
| **MVS (%_{MB})** | 1,99 | 2,03 | 2,00 | 0,002 | 0,00 |
| **pH** | 9,57 | 9,62 | 9,60 | 0,02 | 0,25 |
| **NH₄⁺ (g.kg_{MB}⁻¹)** | 2,13 | 2,27 | 2,20 | 0,08 | 3,48 |

La recirculation d'un ratio mF₁/mF₂ de 30 % de MS traitée par détente flash selon le procédé de l'invention permet dans tous les cas une augmentation de la productivité en méthane de l'invention.

Le dispositif de l'invention permet ainsi d'améliorer la productivité en méthane d'une installation de production de méthane en digestion bi-étagé.

## Revendications

1. Dispositif de traitement d'intrants organiques, de préférence un mélange d'une fraction organique de déchets ménagers, de boues et éventuellement de fumiers et des graisses, le dispositif comprenant :
- Un premier réacteur (110) infiniment mélangé ;
- Un second réacteur (120) infiniment mélangé , ledit second réacteur (120) étant munit d'une boucle de recirculation (130) comprenant une unité de traitement par hydrolyse thermique comprenant un compartiment de traitement par détente flash (132), ladite boucle étant relié d'une part à la sortie du second réacteur et d'autre part à l'entrée du second réacteur,
les premier et second réacteurs (110,120) étant agencés en série.

2. Dispositif selon la revendication 1, dans lequel le premier réacteur (110) est configuré pour fonctionner en conditions thermophiles.

3. Dispositif selon la revendication 1 ou 2, dans lequel le second réacteur (120) est configuré pour fonctionner en conditions mésophiles.

4. Dispositif selon l'une quelconque des revendications précédentes, dans lequel l'unité de traitement par hydrolyse thermique comprenant un compartiment de traitement par détente flash (132) comprend au moins un réacteur de détente flash.

5. Dispositif selon la revendication précédente, dans lequel l'unité de traitement par hydrolyse thermique comprenant un compartiment de traitement par détente flash (132) comprend en outre une presse à vis (131).

6. Procédé de traitement d'intrants organiques, de préférence un mélange d'une fraction organique de déchets ménagers, de boues et éventuellement de fumiers et des graisses, le procédé comprenant les étapes suivantes :
a) introduction des intrants organiques dans un premier réacteur (110) infiniment mélangé,
b) hydrolyse en conditions thermophiles des intrants organiques dans le premier réacteur (110) infiniment mélangé, de manière à obtenir un digestat A ;
c) introduction dans un second réacteur (120) infiniment mélangé du digestat A obtenu à l'étape b) et d'un digestat C obtenu à l'étape e) ;
d) méthanogénèse en conditions mésophiles du digestat A et du digestat C introduits dans le second réacteur à l'étape c), de manière à obtenir un digestat B;
e) séparation du digestat B obtenu à l'étape d) en une première fraction F1 et seconde fraction F2, ladite première fraction F1, optionnellement épaissie, étant soumise à une hydrolyse thermique suivi d'une détente flash de manière à obtenir un digestat C, ledit digestat C étant réintroduit dans le second réacteur (120) à l'étape c).

7. Procédé selon la revendication précédente, dans lequel l'hydrolyse de l'étape b) a une durée allant de 2 à 10 jours, de préférence 5 jours.

8. Procédé selon la revendication 6 ou 7, dans lequel la température d'hydrolyse de l'étape b) est comprise dans un intervalle allant de 50 à 60°C, de préférence 55°C.

9. Procédé selon l'une quelconque des revendications 6 à 8, dans lequel la méthanogénèse de l'étape d) a une durée allant de 10 à 20 jours, de préférence 15 jours.

10. Procédé selon l'une quelconque des revendications 6 à 9, dans lequel la température de méthanogénèse de l'étape d) est comprise dans un intervalle allant de 34 à 40°C, de préférence 37°C.

11. Procédé selon l'une quelconque des revendications 6 à 10, dans lequel la fraction F1 séparée lors de l'étape e) représente 30 à 40 % du volume de digestat A introduit dans le second réacteur à l'étape c).

12. Procédé selon l'une quelconque des revendications 6 à 11, dans lequel l'épaississement de l'étape e) est réalisé dans une presse à vis (131) jusqu'à l'obtention d'une siccité de 10 à 14 %, de préférence de 12%.

13. Procédé selon l'une quelconque des revendications 6 à 12, dans lequel l'hydrolyse thermique suivi d'une détente flash de l'étape e) comprend les sous-étapes :
i) mise sous pression de la fraction de digestat B, de préférence la fraction F1, par injection de vapeur, à une pression allant de 3 à 8 bars, de préférence de 4 à 5 bars, à une température allant de 140 à 180°C, pendant une durée allant de 20 à 40 minutes,
ii) retour à la pression atmosphérique de la fraction de co-digestat B.

14. Procédé selon la revendication précédente, comprenant en outre une étape iii) de transfert de calorie du digestat C pour chauffer la température des intrants, de préférence au moyen d'un échangeur thermique tubulaire.
